Europäisches Patentamt

European Patent Office

Office européen des brevets

(19)

(11) Publication number: **0 083 486**
**A2**

## (12) EUROPEAN PATENT APPLICATION

(21) Application number: **82306736.8**

(22) Date of filing: **16.12.82**

(51) Int. Cl.³: **A 61 K 7/16**

(30) Priority: **01.01.82 IL 64700**

(43) Date of publication of application: **13.07.83**
**Bulletin 83/28**

(84) Designated Contracting States: **CH DE FR GB IT LI NL SE**

(71) Applicant: **Binderman, Itzhak, 13 Fible Street, Tel Aviv (IL)**
Applicant: **Mechanic, Gerald L., 407 Garry Road, Carrboro North Carolina 27510 (US)**

(72) Inventor: **Binderman, Itzhak, 13 Fible Street, Tel Aviv (IL)**
Inventor: **Mechanic, Gerald L., 407 Garry Road, Carrboro North Carolina 27510 (US)**

(74) Representative: **Spencer, Graham Easdale et al, A.A. Thornton & CO Northumberland House 303-306, High Holborn, London WC1V 7LE (GB)**

(54) **Dental care compositions.**

(57) Dental care compositions for inhibiting or eliminating the formation of dental caries, which comprise an effective amount of at least one collagenase inhibitor selected from non-toxic compounds containing thiol groups in free form or in a pharmaceutically acceptable salt or complex form and non-toxic compounds containing disulfide groups; together with a pharmaceutically acceptable carrier to be applied to the teeth and maintained in contact therewith.

- 1 -

Dental care compositions

This invention relates to dental care and oral hygiene compositions having anti-collagenolytic activity owing to which they are useful for inhibiting or eliminating dental caries.

Dental caries is an infectious disease resulting in the progressive destruction of tooth substance, both inorganic and organic. The caries process is initiated by interplay of microorganisms adhering to tooth surfaces and a carbohydrate substrate in contact with a susceptible tooth surface. The caries lesion invariably originates on the external surface of the enamel of erupted teeth or on the exposed cementum of root surfaces and the process then progresses centripetally deep into the dentin. As stated above, dental caries starts by the production of acids through fermentation of the carbohydrate substrate by the microorganisms, and these acids cause demineralization of localized tooth tissue. Thus, the destruction of hard tissues, such as enamel, dentin, cementum or bone, starts with the dissolution of the inorganic material of these tissues, namely hydroxyapatite. In the case of mature enamel this will also result in the decomposition of the organic matrix since this constitutes only 2% of the total composition of enamel. The case is different in other hard tissues (dentin, cementum and bone) which consist of highly cross-linked collagen matrix being resistant to degradation under conditions capable of causing demineralization of those tissues (weak acids or calcium-chelating agents). Native collagen is resistant to general proteolytic digestion under physiological conditions (e.g. by proteolytic enzymes such as trypsin and chemotrypsin) owing to its triple polypeptide helix structure. Collagen can, however, be cleaved by tissue collagenases into two large fragments which can then be further cleaved by non-specific peptidases.

In recent, yet unpublished, studies we have found that dentin material derived from both carious and intact human teeth contains collagenolytic activity, the carious dentin being significantly more active. This fact would indicate that during the tooth development there is produced, besides the collagen, collagenase in an inactive form which may become activated in the course of the carious process and consequently is capable of degrading the collagen matrix of the dentin. Thus, practically the entire collagenolytic activity in the process of the carious disease originates from the collagenase of the dentin itself, although it is known that some bacteria involved in the carious process are able to secrete collagenase enzymes of their own.

It has now been found in accordance with the present invention that the collagenolytic activity of the carious dentin can be inhibited or entirely suppressed by compounds comprising thiol groups, either in the free form (e.g. cysteine) or in latent form, such as in disulfides or in the form of metal salts of the -thio groups. In vivo experiments have shown, in accordance with the invention, that cysteine significantly inhibits the development of carious dentin lesions in rats fed on a cariogenic sugar diet.

The invention thus provides a dental care composition for inhibiting, or eliminating the formation of dental caries which comprises an effective amount of at least one collagenase inhibitor selected from non-toxic compounds containing thiol groups in free form or in a pharmaceutically acceptable salt or complex form, and non-toxic compounds containing disulfide groups; together with a pharmaceutically acceptable carrier to be applied to the teeth and maintained in contact therewith.

The active collagenase inhibitors in accordance with the invention can be selected from non-toxic aliphatic and aromatic mono- and polythiol compounds as well as salts and complexes of the thiol groups in those compounds with pharmaceutically acceptable cations, e.g. alkali metal and alkaline earth metal salts. In addition there may be used, as collagenase inhibitors in accordance with the invention, aliphatic or aromatic disulfides, in which case the dental care composition may optionally comprise a suitable non-toxic reducing agent capable of reducing the disulfide group to form a pair of free thiol groups in situ. Alternatively, a dental care composition according to the invention which comprises a disulfide compound and an auxiliary preparation comprising such a suitable reducing agent, may be applied to the teeth separately, in any sequence.

Further active compounds suitable for use as collagenase inhibitors in accordance with the invention are any edible or other pharmaceutically acceptable substances containing thiol- or disulfide compounds as prosthetic groups. As examples there may be mentioned egg yolk, proteins and protein derivatives, such as hair protein derivatives, and extracts of garlic, onions, scallions, leeks, schallots and the like.

A preferred collagenase inhibitor for use in accordance with the invention is cysteine.

The dental care compositions according to the invention, in particular those wherein the active ingredient comprises free thiol groups, may preferably further include a pharmaceutically acceptable anti-oxidant, for example vitamin C.

The novel compositions according to the invention are adapted for use both in personal everyday oral hygiene as dentifrices (e.g. tooth paste, mouth washes, tooth powders, etc.), as medicated chewing gums and in dentistry. Among the main applications of the compositions in dentistry are the following:

1. sublining and lining of cavities;

2. cavity toilet with the compositions;

3. in indirect pulp capping;

4. in the filling of root canals;

5. for application on any natural abutment before cementation of a bridgework;

6. for application to resorption areas of bone, cementum or dentin;

7. as mouth rinses after periodontal treatment, including surgery.

The specific use for which a dental care composition according to the invention is adapted would determine the appropriate choice of the various parameters of the composition, namely the nature and concentration of the active collagenase inhibitor, the chemical nature and physical properties of the carrier, and the nature of any other additives present, selected from those conventionally used in compositions of this type.

Thus, oral hygiene compositions according to the invention for use as dentifrices should contain the active collagenase inhibitor at a concentration corresponding to from about 1 to about 2000 milli-equivalents of free or latent thiol group per litre of the preparation, more preferably from about 100 to about 1000 milli-equivalents per litre. Thus, for example, when cysteine is used as the active inhibitor in a liquid dentifrice according to the invention, a suitable concentration would be 0.1 molar corresponding to about 1.6% per weight.

Oral hygiene compositions according to the invention for use as dentifrices, may be in the form of powders, pastes or liquids. In addition to the collagenase inhibitors according to the invention these dentifrices will usually also contain the conventional substances. Thus, for example, in a toothpaste there will be incorporated abrasive and/or polishing materials such as calcium carbonate, dicalcium phosphate, calcium phosphate, calcium sulfate or various forms of silica; surfactants, such as sodium lauryl sulfate or sodium dodecylbenzene-sulfonate; gelling agents (or thickeners) such as natural or synthetic gums and gum-like materials, e.g. carboxymethyl cellulose, tragacanth, guar or starch;flavouring and/or natural or synthetic sweetening agents, such as saccharin, flavouring oils (e.g. oils of spearmint, peppermint, wintergreen); and other excipients, such as colouring or whitening agents (e.g. titanium dioxide), preservatives (e.g. sodium benzoate), emulsifying agents and acidifying agents (e.g. phosphoric or citric acid).

Dental care compositions according to the invention in the form of tooth powders and pastes are prepared in the usual manner by mixing the constituents in the dry state or as slurries or solutions.

In general the liquids in the dental pastes or mouth washes in accordance with the invention will comprise primarily water, glycerol, sorbitol or propylene glycol or suitable mixtures of any of these liquids. A mixture of water and glycerol, probably in combination with sorbitol, can be advantageously used.

The invention is further illustrated by the compositions in the following Examples, without being limited thereto in any manner. Quantities are expressed on a weight bases.

Example I

Tooth-paste

| | | |
|---|---|---|
| Precipitated silica (e.g. Neosyl) | about 23 | % |
| Paraffin | 15 | % |
| PAB-esters | 0.2 | % |
| Methylcellulose | 1.8 | % |
| Aromatic substances | 2 | % |
| Cysteine | 1.5 | % |
| Distilled water | up to 100 | % |

Example II

Tooth-paste

| Calcium carbonate | 50 | % |
|---|---|---|
| Tricalcium phosphate | 5 | % |
| Sorbitol (70% solution) | 10 | % |
| Glycerol | 20 | % |
| Tragacanth | 2 | % |
| Aromatic substances | 0.8 | % |
| Cysteine | 2 | % |
| PAB-esters | 0.1 | % |
| Distilled water up to | 100 | % |

Example III

Tooth-paste

| Aluminium-hydroxide | 40 | % |
|---|---|---|
| Na-fluoride | 0.1 | % |
| Sorbitol (70% solution) | 25 | % |
| Glycerol | 5 | % |
| Aromatic substances | 1.2 | % |
| Na-alginate | 1 | % |
| PAB-esters | 0.1 | % |
| Saccharine | 0.25 | % |
| Cysteine | 2 | % |
| Water up to | 100 | % |

Example IV

Tooth-powder

| | | |
|---|---|---|
| Cysteine | 3 | % |
| Aromatic substances | 2 | % |
| Na-cyclamate | 0.5 | % |
| Detergent (Texapon L 100) | 1 | % |
| Calcium phosphate    up to 100 | | % |


Example V

Mouth wash

| | | |
|---|---|---|
| Methylcellulose (low viscous) | 1 | % |
| Aromatic substances | 1 | % |
| PAB-esters | 0.15 | % |
| Dinatrium phosphate 0 aq. | 1.5 | % |
| Citric acid 1 aq. | 1.0 | % |
| Cysteine | 2 | % |
| Distilled water       up to       100 | | % |


Example VI

Tooth-powder

| | | |
|---|---|---|
| Na-cyclamate | 0.75 | % |
| Aromatic substances | 2.25 | % |
| Cysteine | 3 | % |
| Tricalcium phosphate  up to 100 | | % |

Example VII

Tooth-powder

| | | |
|---|---|---|
| Aromatic substances | 1 | % |
| Na-fluoride | 0.1 | % |
| Detergent | 1 | % |
| Cysteine | 2 | % |
| Na-saccharine | 0.25 | % |
| Micro-cystalline aluminium hydroxide | up to 100 | % |

Example VIII

Jet-stream teeth cleaner

To the water to be used in the conventional apparatus to deliver a high velocity jet of water there is added cysteine in the amount of 25 g per litre.

0083486

CLAIMS:

1.     A dental care composition for inhibiting or eliminating the formation of dental caries, which comprises an effective amount of at least one collagenase inhibitor selected from non-toxic compounds containing thiol groups in free form or in a pharmaceutically acceptable salt or complex form and non-toxic compounds containing disulfide groups; together with a pharmaceutically acceptable carrier to be applied to the teeth and maintained in contact therewith.

2.     A composition according to Claim 1 comprising, as collagenase inhibitor, a non-toxic aliphatic or aromatic thiol.

3.     A composition according to Claim 2, further comprising vitamin C.

4.     A composition according to Claim 1 comprising, as collagenase inhibitor, a non-toxic aliphatic or aromatic disulfide.

5.     A composition according to Claim 4, further comprising a non-toxic reducing agent capable of reducing a disulfide group to yield a pair of thiol groups.

6.     A composition according to Claim 2 or 3 comprising cysteine as the collagenase inhibitor.

7.     A composition according to any one of the preceding claims comprising said collagenase inhibitor in an amount corresponding to from about 1 to about 200 milliequivalents of free or potential thiol group per litre of the composition.

8.     A composition according to Claim 6 comprising said collagenase inhibitor in an amount corresponding to from about 100 to about 1000 milliequivalents of free or potential thiol group per litre of the composition.

9.     A composition according to any one of the preceding claims, adapted for use as a dentifrice, in the form of a toothpaste, tooth powder, mouth wash or a high velocity water jet.